# EUROPEAN PATENT SPECIFICATION

(11) **EP 3 305 461 B1**
(45) Date of publication and mention of the grant of the patent: **13.05.2020**
(21) Application number: 16192271.1
(22) Date of filing: 04.10.2016
(51) Int. Cl.: B23K 26/362, B23K 26/402, G01N 33/38, G01N 21/87, H01J 49/04, B23K 103/00

(54) **SYSTEM FOR MARKING AND ANALYSING GEMSTONES**
SYSTEM ZUR MARKIERUNG UND ANALYSE VON EDELSTEINEN
SYSTÈME DE MARQUAGE ET D'ANALYSE DE PIERRES PRÉCIEUSES

(43) Date of publication of application: 11.04.2018
(73) Proprietor: Swiss Gemmological Institute SSEF, 4051 Basel (CH)
(72) Inventor: Wang, Hao, 8003 Zürich (CH); Krzemnicki, Michael S., 4054 Basel (CH)
(74) Representative: BOVARD AG

(56) References cited:
- WO-A1-2015/103492
- US-A1- 2003 019 852
- US-A1- 2003 038 121
- US-B1- 7 557 917
- EREL E ET AL: "CAPABILITIES OF LASER ABLATION MASS SPECTROMETRY IN THE DIFFERENTIATION OF NATURAL AND ARTIFICIAL OPAL GEMSTONES", ANALYTICAL CHEMISTRY, AMERICAN CHEMICAL SOCIETY, US, vol. 75, no. 23, 1 December 2003 (2003-12-01), pages 6422-6429, XP001047346, ISSN: 0003-2700, DOI: 10.1021/AC034576T

## Description

The invention relates to a system for handling gemstones, comprising a micro-inscribing system for inscribing information onto a surface part of a gemstone using an ablation process and further comprising a material analysing system for analysing the contents of a material composition. The invention further relates to a method for handling a gemstone, wherein the handling comprises at least a marking of said gemstone by an ablation process and an analysis of the material composition of said gemstone.

In the field of gemstones, the price of a gemstone not only depends on its type (for example whether it is an emerald, a diamond, a ruby, a sapphire or the like) and on its weight (typically measured in carat = ct), but also severely on other factors like the quality of the stone (whether it shows some inclusions or impurities or not), its colour (due to contaminations with some elements) and even its origin (i.e. where it has been mined). Since at least some of these factors can only be evaluated by an expert (possibly necessitating the use of sometimes quite costly analysing instrumentation), it has become common in the meantime to inscribe some information onto the gemstone itself that can be read and (at least to some extent) understood even by an unskilled person. Due to the limited size of gemstones, and due to the fact that the gemstone should not be negatively affected by such an inscription (marking), the respective inscription should be as small as possible so that usually a loupe will be needed for reading the information. However, such loupes are inexpensive and widely available.

It has been suggested in the state of the art to use markings like a brand, a trademark or a proof of origin (this is information which is sort of static and common to a plurality and variety of gemstones). However, it has also been proposed in the state of the art to use individual markings as well, like information about the origin of the stone, its weight and the like. Even the use of some reference information (for example a unique gemstone identification number, which is some kind of a serial number) so that the respective information can be retrieved in detail from a computer database and/or the use of cryptographic methods to hinder any fraud have already been proposed. An example for this can be found in US laid open document US 2003/0019852 A1. Usually, for generating such inscriptions, laser techniques are commonly employed.

As already mentioned, an evaluation of some of the characteristics of the stone quite often necessitates the use of elaborate instrumentation. Just as an example, the origin of a stone can usually only reliably be verified if the material composition is measured (presence and weight percentage of certain elements and sometimes even of their isotopes). Another problem is that gemstones already have been altered with criminal intent, for example by doping a gemstone with certain elements to alter its colour. To detect such alterations, an analysis of the material composition is quite often unavoidable as well.

For evaluating gemstones in this respect, the use of mass spectrometry is already known in the state of the art, see for example Erel E. et all, "CAPABILITIES OF LASER ABLATION MASS SPECTROMETRY IN THE DIFFERENTIATION OF NATURAL AND ARTIFICIAL OPAL GEMSTONES", Analytical chemistry, American chemical society, vol. 75, no. 23 of 1 December 2003, pages 6422-6429. In particular, so-called inductively coupled plasma mass spectrometry (ICP-MS), even more particularly so-called inductively coupled plasma time-of-flight mass spectrometry (ICP-TOF-MS) is known in the state of the art.

As it is clear for a person skilled in the art, the material (i.e. that of the gemstone) to be analysed has to be provided in a way that it becomes suitable for the measuring method in question. In particular when it comes to gemstones, it is of course desired to use as little material as possible, in particular for monetary and aesthetic reasons. For this, laser ablation techniques have already been employed, in particular in connection with mass spectrometry methods. Nevertheless, despite the fact that only a small amount of material has to be used for this, there is nevertheless a need to remove some material of the gemstone to be able to perform the measurement; something that is of course undesired. Therefore in quite some cases analysing methods that do require more costly apparatuses and/or whose preciseness and validity is inferior as compared to mass spectrometry methods are still employed despite of their disadvantages - namely for the reason that they do not require to remove a material sample from the gemstone.

Therefore there is still quite some room for improvements when it comes to the handling of gemstones.

It is the object of the invention to provide a system for handling gemstones that is improved over systems for handling gemstones that are known in the state of the art. It is another object of the invention to propose a method for handling gemstones that is improved over methods for handling gemstones that are known in the state of the art.

The present invention, namely a system according to independent claim 1 and a method according to independent claim 9 solve these objects.

It is therefore suggested to design a system for handling gemstones that comprises a micro-inscribing system for inscribing information onto a surface part of a gemstone using an ablation process, where the system further comprises a material analysing system for analysing the contents of a material composition in a way that the material that is set free by said micro-inscribing system when inscribing information onto a gemstone is used by said material analysing system for analysing the material composition of the gemstone. In principle, for the micro-inscribing system essentially all possible methods of performing an ablation process can be envisaged. In particular, for this a light beam (laser beam, x-ray beam, focused light beam and so on) and/or a particle beam (ion beam, electron beam or the like) can be used. However, typically the use of a shortwave laser beam is preferred, in particular because such lasers are commercially available on the market, are usually comparatively cheap and fulfil the requirements pretty well. In particular, it has to be noted that the ablation process (for example when using a laser) should be performed in a way that the gemstone does not melt due to the power input. Instead the power input should result in small explosions near the surface of the gemstone, so that some solid particles are blown off the surface, which is known as an ablation process. By this, small craters are generated, where the material that was formerly present in the crater is removed in form of small solid particle chips (where the solid-state particles can be later on treated in a way to make them suitable for the following analysing method, for example by vaporising and/or ionising them in particular by using inductively coupled plasma methods for mass spectrometry). Likewise, the material analysing system can be of essentially any type. In particular, it is advantageous in the context of the present invention if said material analysing system necessitates a certain amount of material in form of a sample for analysing purposes. Of course, it is possible that such a destructive analysing method is only part of the overall analysing process, so that additional analysing methods (including both destructive and/or non-destructive methods) are used for analysing the gemstone. While both methods, i.e. the micro-inscribing method and the material analysing method, as well as the systems that are used for performing those methods are known in the state of the art as such, it is presently proposed to combine the two methods in an advantageous way, so that advantageous effects result. The idea is to use the unavoidable ablation of material when inscribing markings in a way that the thus generated "waste material" is not simply discarded, but instead used as a "valuable input material" for the material analysing system/method. Using this simple idea, it is quite often possible to employ essentially all known methods for material analysis (including mass spectrometry methods) without any material loss of the gemstone that goes beyond the amount that is necessary for marking. Of course, at least in certain situations it is both possible that the material that is ablated in the course of the micro-inscribing process exceeds the amount of material that is necessary for analysing the material composition. Then, the excess amount of material can be simply discarded. Likewise, it might prove to be necessary to ablate some more material than would be needed for marking the gemstone. Nevertheless even then the presently proposed system/method is advantageous since the amount of material to be ablated for analysing purposes will be reduced, typically at least quite considerably.

It is further suggested that the micro-inscribing system of the presently suggested system for handling gemstones is designed in a way to inscribe information, at least in part, in form of a series of points, in particular in form of a series of raster points. Instead of "points", one could also talk about "pixels". By using a series of points, typically the amount of material that has to be ablated from the gemstone can be further reduced, in particular as compared to inscribing methods, where continuous lines are inscribed. This is, because when looking at the inscribed information, the eye (or a scanner) will "complete" those parts between the individual points that would be removed when using a continuous line. Thus, the remaining "walls" do not have to be removed. Another advantage is that an ablation of the material of the gemstone in form of individual points will usually improve the ablation process and/or will improve the usability of the ablated material with respect to analysing methods, in particular with respect to mass spectrometry methods (although other methods can prove to be useful as well). Even more, for certain kinds of inscribed information, a marking in form of several points might be advantageous for technical reasons and/or for readability as well. In particular, if the information is of a digital nature and intended to be read out by a computer system (like a QR-code, using a QR-code scanner), this statement is particularly true. When talking about "raster points", it is meant that the individual points can only be placed at discrete positions according to a certain grid/matrix. However, one can also place points deviating from such a fixed grid/matrix, if this should be advantageous for the concrete problem. It should be noted that the inscribed information is typically of a size that it is not readily visible to the naked eye. Therefore, considerations of aesthetics do play a (relevant) role; in particular, the marking of the stone should preferably be done in a way that it is hardly visible by the naked eye for aesthetics reasons.

A particularly advantageous embodiment of the presently proposed system for handling gemstones can be realised if the micro-inscribing system of the system for handling gemstones is designed in a way to inscribe information, at least in part, as a 2-D code, in particular as a data matrix code or as a QR-code. Additionally or alternatively, the 2-D code preferably has a size of not more than 1 mm x 1 mm, preferably of not more than 0.5 mm x 0.5 mm, even more preferred of not more than 0.3 mm x 0.3 mm. However, different values (essentially) and/or upper limits are possible as well like 0.75 mm x 0.75 mm, 0.4 mm x 0.4 mm, 0.2 mm x 0.2 mm or 0.1 mm x 0.1 mm. If it should be noted that the presently suggested embodiment can be realised particularly well by way of a point-like representation (pixel-like representation). When using a 2-D code, a comparatively large amount of information can be inscribed, albeit a very small surface area has to be used for this. Typically some 50 characters of data contained in the 2-D code is considered to be a sensible minimum. Using this amount of characters, an information like "SSEF88888, Sapphire, Kashmir, 10.000ct" can be inscribed (where SSEF88888 is the gemstone's unique identification number; this number can also be used for retrieving more information about the gemstone from a database later in time, including an online database). Of course, a larger amount of data is possible as well. Furthermore, it should be noted that albeit presently 2-D codes are typically of a square shape, a rectangular shape, showing a different length and height (or even other geometrical forms) can be employed as well. Therefore, the indicated sizes could be "intercombined" as well, so that for example a rectangular 2-D code of a size of not more than 1 mm x 0.5 mm is considered to be disclosed as well. Although it is possible that the marking of the gemstone is a 2-D code only, some additional information might be included as well. As an example, the most important information about the gemstone could be repeated in standard characters (for example Latin characters, Hebrew characters, Chinese characters, Japanese characters or the like) and/or a logo of the company might be added.

It is further suggested to design the system for handling gemstones in a way that the system further comprises an information generation system that is able to actuate said micro-inscribing system, in particular in a way that individual information per gemstone can be inscribed. Of course, some information might be repeated for all stones (for example a brand logo or the like) or for a certain larger number of gemstones (like the indication "diamond" or the like). However, preferably at least part of the information is individually and uniquely applied for the very gemstone in question, so that a unique identification can be realised (including the possibility of retrieving additional information from a database later on). This unique identification can be the already described gemstone identification number. A unique identification can reduce the possibility for fraud. It is even possible that cryptographic methods like a digital signature or the like will be used for the information. The information generation system can be an digital computer (including, but not limited to personal computers, workstations, single board computers, electronic controllers and the like). The output can be used to actuate certain parts of of the micro-inscribing system, for example the movement of stepper motors that influence the direction of the laser beam, the modulation of the laser beam (on/off), a movement of a support for the gemstone (where the support might be designed in a way that it can hold a plurality of gemstones as well and so on). Of course, such an actuation of actuators can be used as well, if "non-unique information" (information that is common to at least several gemstones, if not essentially all) is to be inscribed.

Another preferred embodiment of the presently proposed system for handling gemstones can be realised if the micro-inscribing system comprises a laser, in particular a laser with a short wavelength of not more than 266 nm, of not more than 213 nm or of not more than 193 nm and/or if the micro-inscribing system comprises a focusing system for focusing the outputted laser beam of said laser, preferably a focusing system for focusing the size of the laser beam spot to a size of 0.5 to 50 µm, 1 to 30 µm, 2 to 25 µm or 5 to 15 µm, preferably to 10 µm. Of course, the given upper and/or lower values and/or limits of the indicated intervals can be interchanged, so that, for example, an interval from 1 to 10 µm or 2 to 15 µm is considered to be disclosed as well. Additionally, different values and/or upper limits for the wavelengths could be employed as well, like 300 nm, 250 nm, 200 nm, 150 nm, 100 nm or the like. First experiments have indicated that the presently indicated numbers are particularly advantageous and thus should be employed. In particular, additionally and/or alternatively the micro-inscribing system may comprise a laser beam shaping device, which can be present in form of an aperture or the like. This way, the laser spot can have a square shape (which is usually preferred), or any other shape like a rectangle, a circle, a triangle, an ellipse and so on.

Another preferred embodiment of the system for handling gemstones according to the present invention can be realised, if said laser of said micro-inscribing system is a pulsed laser, preferably a laser with a repetition rate of 1 to 100 Hz, preferably of 5 to 50 Hz, even more preferred of 10 to 25 Hz, in particular 20 Hz and/or if the laser of said micro-inscribing system is a high-power laser with the laser fluence of not less than 0.5 J/cm², preferably of not less than 1 J/cm², more preferred of not less than 2 J/cm², even more preferred of not less than 3 J/cm². However, even higher laser fluences may be used as well, for example laser fluences of essentially and/or not less than 5 J/cm², of essentially and/or not less than 7 J/cm² or even of essentially and/or not less than 10 J/cm². Again, it should be noted that the upper and lower limits of the intervals as given can be interchanged. When using a laser with one of the aforementioned features, preferably with both features, the inscribing process can be realised in a particularly advantageous way. As an example, the repetitive nature of the ablation process will enable to employ a still comparatively weak and thus typically comparatively cheap laser, while the time for applying the mark is still limited to a decent time, like in the 10 to 30 minute range (for a complete marking of one gemstone). In particular with the suggested fluences, a good ablating behaviour of the micro-inscribing system can result, so that the ablated material is particularly suitable for being analysed with respect to its material composition. In particular, an (undesired) melting of the gemstone can be avoided. Only for completeness it is mentioned that with the fluences given, a typical crater (usually a depth of 15 µm to 20 µm is desired for the crater) cannot be created with a single laser shot. Instead, a plurality of laser shots is necessary. A number of laser shots in the order of 40 for a single spot/pixel/crater is somewhat typical.

It is even more preferred to design the system for handling gemstones in a way that the material analysing system comprises a mass spectrometry system, preferably an inductively coupled plasma mass spectrometry system (ICP-MS), more preferred an inductively coupled time-of-flight mass spectrometry system (ICP-TOF-MS) and/or an inductively coupled plasma quadrupole mass spectrometry system. When using such mass spectrometry systems, in particular using mass spectrometry systems of the aforementioned type, a particularly valid information for determining information with respect to important features of gemstones can be realised. As an example, using the information that can be obtained by mass spectrometry systems, one can determine the origin of the gemstone in question and/or discover any fraudulent manipulations of the gemstone (for example doping the gemstone with certain elements to change its colour). When using inductively coupled plasma systems for the mass spectrometry system, the ablated material will be processed in a way that it becomes particularly suitable for mass spectrometry systems. Moreover, when employing time-of-flight mass spectrometry systems, the measurement quality will usually increase even further. Another thing that should be mentioned is that by using certain types of mass spectrometry methods, in particular time-of-flight mass spectrometry methods, the repetitive ablation of material and thus the pulse-like occurrence of material to be analysed, will surprisingly not (significantly) negatively influence the measurement resulta. So far, in the state of the art there was a prejudice against using a pulse-like flow of material for mass spectrometry systems. It was believed that the measurement quality will be degraded in a significant and - even more problematic - not foreseeable away. The inventors were surprised to see that this is not the case, in particular when using inductively coupled plasma systems and even more particular in combination with time-of-flight mass spectrometry. It seems that this prejudice comes from the observation that when using mass/charge-spectrometry measurements (so-called Q-MS), the measurement results are indeed quite often bad and sometimes even not usable.

Furthermore, it is suggested to design the system for handling gemstones in a way that said material analysing system is designed in a way to analyse material that is preferably contained in a carrier gas, wherein said carrier gas is preferably an inert gas, taken from the group comprising helium, nitrogen, neon, argon, krypton, xeon and so on. This way, it is possible to transport ablated material from the point, where the marking of the gemstone is performed, to the analysing apparatus. It should be noted that the ablated material is normally contained in the gas stream of the carrier gas in form of small solid particles (generating a smoke of carrier gas and ablated material). It should be noted further that this combined stream of ablated material and carrier gas can be processed before the actual measurement is taken (typically, this is even preferred). For example, this can be done with inductively coupled plasma systems, where by virtue of the plasma any solid particles are vaporised and/or ionised, so that the material becomes more suitable for mass spectrometry analysing methods. The carrier gas can be a flushing gas for a sample chamber (wherein one or several of the gemstones to be handled can be contained). The sample chamber can comprise one or several suitably sized windows (which are transparent for the wavelength of the laser light in question), so that the laser beam can enter the sample chamber.

Furthermore, a method for handling a gemstone is suggested, where the handling comprises at least a marking of said gemstone by an ablation process and an analysis of the material composition of said gemstone in a way that the material of the gemstone that is set free by the ablation process is used for analysing the material composition of the gemstone. The typical features and advantages that come forth with the presently suggested method are similar to the advantages and features of the already described system for handling gemstones, at least an analogy. Therefore, for brevity these features and advantages are not repeated in full detail again. Furthermore, it is of course possible to modify the presently suggested method in a way that was already described in the context of the presently suggested system, at least in analogy.

In particular, it is possible to employ the method in a way that the ablating process is performed by a laser beam, preferably using a spotwise ablation of the gemstone, wherein material that is ablated in a spotwise manner is used for analysing purposes. The details of this embodiment have already been described in the context of the presently suggested system, at least in analogy.

Another possible embodiment of the method can be realised, if a cleaning ablation step is performed prior to an analysis ablation step, where only the material that is ablated during the analysis ablation step is used for analysing the material composition of the gemstone. This can be done by using a "two-step process" for making the mark, for example. In a first step (the cleaning ablation step), a thin layer of material (of possibly one 10^{th}, 1/5, one quarter or one third of the required depth for a pixel/crater of the marking) is ablated. The material that is ablated during this cleaning ablation step (typically a set of ablation processes/laser shots) is simply dumped (and in particular not used for analysing purposes). This takes into account the possibility of external impurities that are possibly present and which are typically limited to the very upper surface parts of the gemstone. Such impurities can stem from the genesis of the gemstone, but also from material that is used for grinding the gemstone and so on. Nevertheless, such impurities can be disadvantageous for the validity of the analysing process to be performed. Then, in a second step, the already partially present pixel-like cavity (crater) will be made deeper by a second ablation step (typically a set of ablation processes/laser shots; one could talk about an analysing ablation process). This way, the marking will be completed, but this time the ablated material will be analysed, in particular with respect to its material composition. Now, external impurities are only present at a reduced level (if at all). The separation into a cleaning ablation step and an analysis ablation step can be done in a way that for every position (or a group of individual positions; pixels) first a cleaning ablation step and afterwards an analysis ablation step is performed. However, for performance reasons and (typically) for improving the quality of the measurement as well, the complete marking (or at least an essential part of it) is first inscribed partially using a cleaning ablation step and is afterwards completed in a second run using an analysis ablation step. Also, it is to be mentioned that typically the depth that has to be used for a crater so that a usable (readable) marking will result will typically be reached only after a plurality of laser shots (ablation steps). A typical number for this is 40 laser shots, thus creating a typical depth of 18 µm. Then, the cleaning ablation step could comprise 10 laser shots, while an analysing ablation step could comprise 30 laser shots (as an example). Nevertheless, it should be noted that in a number of cases (in particular dependent on the gemstone and/or the type of gemstone in question) a cleaning ablation step is not necessary/favourable, since it does not significantly improve the quality of the analysis of the material. Then, a single ablation step, in other words an "exclusive analysis ablation step" can be performed as well. In particular, the above suggested system for handling gemstones can be designed in a way that it can perform one or both of the suggested methods (cleaning and analysis ablation step and/or analysis ablation step only).

Another preferred embodiment of the presently suggested method can be realised if the analysis of the material is at least in part performed by using mass spectrometry methods, in particular inductively coupled plasma mass spectrometry methods, preferably inductively coupled plasma time-of-flight mass spectrometry methods and/or for inductively coupled plasma quadrupole mass spectrometry methods. This has already been describedin the context of the presently suggested system for handling gemstones, at least in analogy.

Likewise, the method can be employed in a way that the marking of the gemstone, in particular the marking of the gemstone that is performed in a spotwise manner, comprises a 2-D code, in particular a data matrix code or a QR-code. Again, it is referred to the comments that were already made with respect to the suggested system.

Even more, it is suggested to employ the method in a way that the marking varies at least in part with the marked gemstone. Again, reference is made to the previously given description, at least an analogy.

Further advantages, features, and objects of the invention will be apparent from the following detailed description of the invention in conjunction with the associated drawings, wherein the drawings show:
Fig. 1 a possible embodiment of a system for marking and analysing gemstones in a schematic overview;
Fig. 2 a possible 2-D data marking that was applied using the system according to Fig. 1;
Fig. 3 a typical measurement signal that can be obtained when employing the system according to Fig. 1.

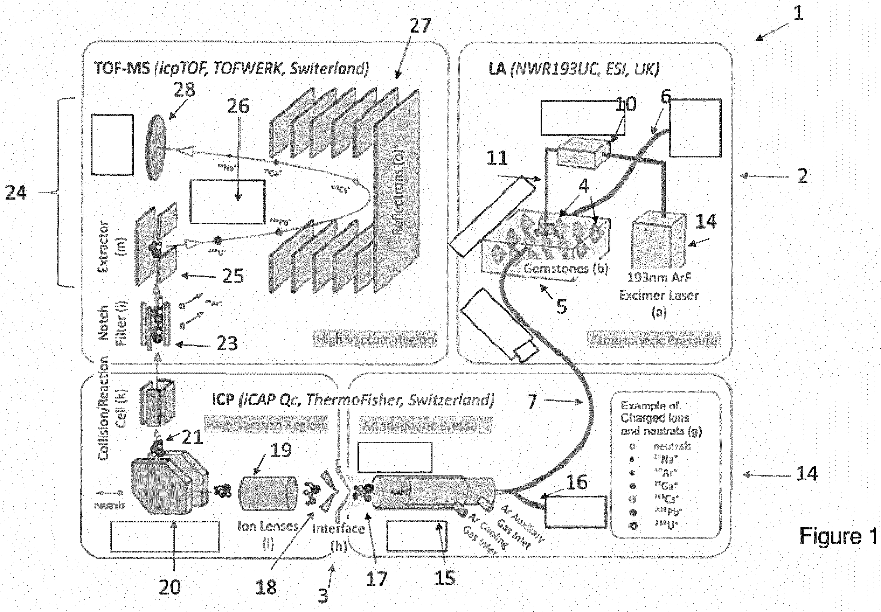

Fig. 1 shows a possible embodiment of a system for handling gemstones, namely for inscribing a marking onto a gemstone and, at the same time, for analysing the chemical content of the gemstone using mass spectrometry methods, in a schematic overview.

The detailed designs of the various components used are as such known in the state of the art and are therefore not further elucidated in the present context for brevity.

The overall system 1 for handling gemstones comprises two main groups, namely the information inscribing part 2 and the material analysing part 3.

In the information inscribing part 2, a plurality of individual gemstones 4 is arranged in a sample chamber 5. The sample chamber 5 is constantly flushed by a carrier gas (usually an inert gas, presently helium). The carrier gas is introduced into the sample chamber 5 via a gas inlet hose 6, passes by the gemstone 4 and leaves the sample chamber 5 through the gas outlet hose 7. When leaving the sample chamber 5, the carrier gas typically comprises (at least during part of the time) some small solid-state particles, namely particles that were set free from a gemstone 4 by laser ablation techniques, and are carried along together with the carrier gas. Therefore the outgoing carrier gas is at least at times some kind of a smoke (small solid particles contained in gas). This will become clearer in the following.

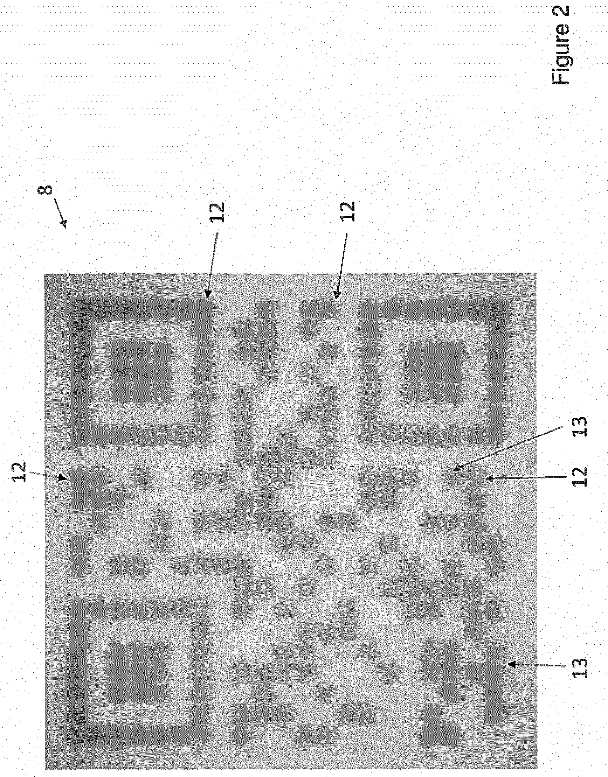

As it is quite common in the meantime, the gemstone 4 will be inscribed with a marking, which is presently applied in form of a 2-D data code, namely a so-called QR-code 8 (which is known in the state of the art as such). An example for such a QR-code 8 is shown in Fig. 2. The exact design of the QR-code 8 will be described in more detail in the following.

The inscribing process of the gemstones 4 is performed using laser ablation techniques. For this, a high-power laser 9 is used. Presently, a ArF excimer laser with a short wavelength of 193 nm is used. However, lasers with a somewhat longer wavelength can be successfully used in the context of the present invention as well. In particular, successful experiments were performed with lasers having a wavelength of 213 nm and 266 nm, as well.

The present laser 9 has a laser fluence of approximately 5 J/cm². Using appropriate laser optics 10, the diameter of the laser beam 11 was focused to a square laser spot of 10 µm x 10 µm in size. Therefore, the laser optics 10 contains some apertures as well. However, it should be noted that round laser spots or different geometrical shapes for the laser spot can be employed as well. The laser 9 is of a repetitive type with a laser repetition rate of presently 20 Hz.

The laser beam 11 enters the sample chamber 5 through a window (presently not shown; several windows could be used as well) that is transparent for laser light of the wavelength in question (which does not necessarily mean that it is transparent for light taken from the visible light spectrum).

During every shot, a certain, small part of the gemstone 4 (which is approximately equivalent to the 10 µm x 10 µm size of the focused laser beam 11) is ablated. This means that due to the energy input (essentially because of the thermal effects) small solid particles will explode away from the surface, where they are taken up and carried along by the inert carrier gas that is introduced through inlet hose 6 to the sample chamber 5, leaving the sample chamber 5 afterward via gas outlet hose 7. Information is inscribed by moving the relative position of the gemstone with respect to the current position of the laser spot. This can be done either by moving the sample chamber 5 (for example by using a movable table that is actuated by stepper motors and on which the sample chamber 5 is placed), or by offsetting (bending) the focused laser beam 11.

The system (typically the complete system 1, comprising the information inscribing part 2 and the material analysing part 3) is controlled by a digital computer (not shown) which also receives and stores the measured data, as described later on. To generate craters on the surface of the gemstone 4 in a way that the inscribed information can be retrieved in a well readable way, the craters have to have a size in the order of 10 µm x 10 µm and a depth of some 15 to 20 µm in depth (typically around 18 µm). Given the current performance of the laser 9 as described, this means that a single shot per pixel 12 is not sufficient to generate a sufficiently deep crater. Therefore, a series of several laser shots has to be applied. A typical number for this is around 40 shots per crater.

Once a crater is "finished", the ablation point 12 is changed to the next position.

An alternative - and even preferred - way of applying the data is to first apply a series of typically 10 laser shots per pixel in a first run, thus generating a hardly readable marking on the gemstone 4 (even when using a loupe). The material that is ablated from the gemstone 4 during this session is simply dumped and not used for analysing (as discussed in the following). Therefore, this first part of the inscription process could be understood as a surface cleaning process. Since impurities in the gemstone's material are normally confined to the very upper layers of the gemstone 4, this means that surface parts that are significantly contaminated with impurities (if present) are removed from the surface without causing any questionable measurement results. Only during the second round of inscribing the markings, the material that is ablated in the course of the inscribing process is used for analysing purposes. With the above indicated numbers, material that is ablated in the course of 30 laser shots will thus be used for analysing purposes. However, the fraction between cleaning ablation shots and analysing ablation shots can be chosen to be different, of course.

Another feature of the presently proposed inscribing process is that the relative movement between the focused laser beam 11 and the gemstone 4 when changing from one spot 12 to the neighbouring one, is set to a value that is somewhat larger than the diameter of the laser spot of the focused laser beam 11. In the presently shown embodiment, the relative movement is chosen to be 15 µm (50% more, to be more general; however, different offsets could be used as well, like 10% more, 25% more, 75% more or even 100% more). This can be clearly seen from the finished QR-code 8 (see Fig. 2), where some residual walls 13 are visible between 2 neighbouring pixels 12.

This is not problematic for reading. On the contrary: given the small dimension of the QR-code 8 (presently the size of the finished QR-code 8 is 0.3 mm x 0.3 mm), the presently shown QR-code 8 is better readable by electronic means (possibly after optical magnification) as opposed to a "continuous QR-code".

Only for completeness, it should be mentioned that additional information apart from the QR-code 8 as shown in Fig. 2 can be applied as well. As an example, a logo, latin characters or something similar can be applied as well, in particular in addition to the QR-code 8. Of course, instead of a QR-code 8, a data matrix code or another type of 2-D code can be equally employed.

As it is clear, applying an informative marking on a gemstone 4 is a (slightly) destructive process. Despite of this, the application of such markings has not only become tolerated in the meantime, but is quite often desired by the consumer, or even mandatory for legal requirements. This is because the gemstone 4 can be uniquely identified; therefore its history, in particular its origin, can be identified, possibly by looking up the respective information by means of a database.

So far, the material that was removed (ablated) during the information inscribing process was simply dumped.

Presently, however, it is suggested to use this ablated material for some additional sensible purpose, namely for performing a material composition analysis, using the material analysis part 3 of the presently proposed system 1. This idea is advantageous, because beforehand, when employing mass spectrometry methods, a material sample was removed from the gemstone 4 solely for this purpose - leaving a small crater, having a negative influence on the surface of the gemstone 4.

Therefore, the ablated material that was ablated in the information inscribing part 2 is flushed out of the sample chamber 5 through gas outlet hose 7 by means of the carrier gas (presently helium). Therefore, the gas in gas outlet hose 7 is some kind of a smoke, namely the carrier gas containing small particles of solid, ablated material.

This material mixture is further refined in the initial part of the material analysis part 3, before the actual spectrometric measurement takes place, namely in the refinement part 14.

The refinement that takes place in the refinement part 14 is initially performed by an inductively coupled plasma torch (ICP-torch 15). Such ICP-torches 15 are, as such, known in the state-of-the-art. First of all, the incoming gas is mixed with a nebuliser gas through nebuliser gas inlet 16, shortly prior to the ICP-torch 15. As the analyser gas, Argon is presently used. Argon has the advantage (similar to helium) that it is chemically inert and it is not present (at least to a significant amount) in naturally occurring gemstones 4. Therefore, undesired noise signals during measurement can be avoided.

In the ICP-torch 15, the gas mixture/smoke is heated to a very high temperature, so that a plasma 17 is created (by vaporising and ionising the solid-state particle chips), leaving the ICP-torch 15 through an orifice. The plasma beam 17 contains ions of the various contained atoms, typically at differently charged states. The plasma beam 17 passes through an interface 18, which can be an arrangement of a plurality of serially aligned small holes. The interface 18 is a good barrier against the penetration of gas, which is necessary since the part with the ICP-torch 15 is under atmospheric pressures (right part of refinement part 14 in Fig. 1), while the following components are under high vacuum (left side of refinement part 14 in Fig. 1).

The thus extracted fraction of the generated plasma beam 17 is further processed by several components, namely some ion lenses 19, where the (ionised fraction) of the plasma beam 17 is focused. The plasma beam 17 then passes through an angular deflection lens 20, where neutral particles are removed from the plasma beam 17. Furthermore, higher charged ions are usually removed as well, at least to a certain extent (different deflection angle). The remaining ion beam part 21 of the plasma beam 17 passes through a so-called collision cell 22, where polyatomic interferences can be reduced. Now, the ion beam 21 is led through a notch filter 23, where the Argon ions (Argon was introduced as a nebuliser gas through nebuliser gas inlet 16) are removed.

The remaining ion beam 21 then passes through a time-of-flight mass spectrometer 24 (TOF-MS), a system that is as such known in the state of the art.

Just to give a very short idea about the functionality of a TOF mass spectrometer 24: the ion beam enters an extractor 25, which pushes ions at a high frequency into the field free time-of-flight region 26. Ions are mirrored ("bounced back") by a reflectron 27 and finally registered by a detector 28.

It should be noted that instead of a time-of-flight mass spectrometer 24, another type of (mass) spectrometer can be used as well. Preferably, the (mass) spectrometer should be able to not only measure the ratio between charge and mass, because then some ambiguities could arise in the measured signal, leading to a decreased significance of the measured data.

The measured data can be stored in a computer database and can be linked with the unique identification number that was inscribed onto the gemstone 4 in the information inscription part 2 of the system 1. This information can be accessed and retrieved by an interested person later on, when he wants to check some properties of the gemstone 4 in the future.

A possible example for a QR-code 8 is shown in Fig. 2. Fig. 2 acutally shows a picture of a QR-code 8 that was applied in an experimental setup of a system 1 according to Fig. 1.

As can be seen in Fig. 2, the QR-code 8 comprises a plurality of specially arranged pixels 12. A QR-code 8 is particularly well recognisable by the solid squares that are surrounded by rectangular frames in 3 of the 4 corners of the QR-code 8. The size of the presently shown QR-code 8 is 0.3 mm x 0.3 mm. The example could read "SSEF88888" which is the identification code for this particular gemstone 4. If the data matrix of the QR-code 8 is chosen somewhat larger, it is possible as well to include some directly retrievable information, like "SSEF88888, Sapphire, Kashmir, 10.000ct" (as it is presently the case), so that some information can be obtained even without looking up in a database, which might be helpful under certain conditions.

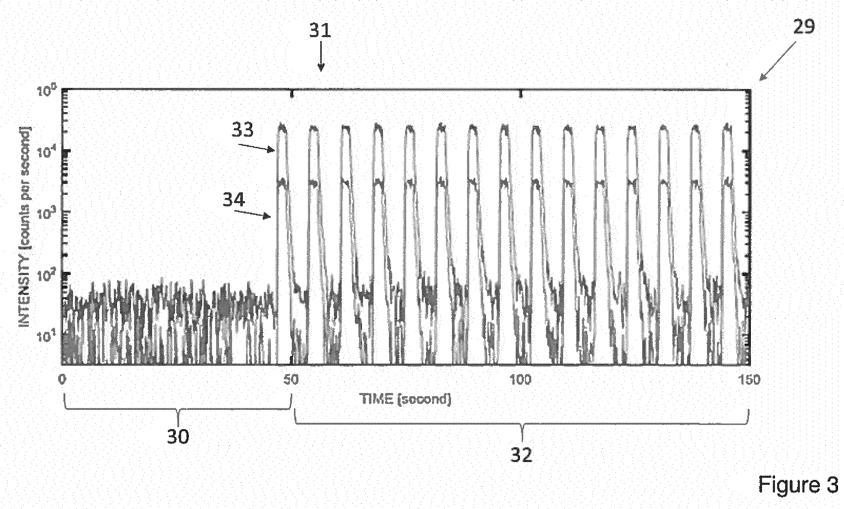

Finally, in Fig. 3, a transient signal 29 that was retrieved by an experimental setup of a system 1 for handling gemstones 4 according to Fig. 1 is shown. While the complete transient signal 29 is longer, presently only a zoom-in of the first 150 seconds of the complete signal is shown (the complete transient signal 29 has a length of some 1.800 seconds = 30 minutes).

In the initial phase of the transient signal 29, some noise signal 30 is visible. Here, the ablation has not yet started. After 50 seconds, the ablation process, namely a repetitive ablation of the gemstone's 4 surface in form of individual pulses of a laser beam 11, has started. The individual pulses 31 can be clearly seen in the data containing part 32 of the transient signal 29.

Furthermore, the individual peaks 31 show two individual signals 33, 34 each, which can be clearly separated from each other. The two visible peaks 33, 34 are signals for 27AI 33 (aluminium concentration 2%wt) and 69Ga 34 that is contained in the gemstones 4. As can be seen, the ratio between the 2 signals 33 and 34 (27AI and 69Ga) is essentially stable in the course of all individual peaks 31. Therefore, it is clear that this is a very valid and reproducible signal, even when the material comes in as a series of individual pulses.

**Reference List**

| | | | |
|---|---|---|---|
| 1. | system for handling gemstones | 18. | interface |
| 2. | information inscribing part | 19. | ion lens |
| 3. | material analysing part | 20. | angular deflection lens |
| 4. | gemstone | 21. | ion beam |
| 5. | sample chamber | 22. | collision cell |
| 6. | gas inlet hose | 23. | notch filter |
| 7. | gas outlet hose | 24. | TOF mass spectrometer |
| 8. | QR-code | 25. | extractor |
| 9. | laser | 26. | field free time of flight region |
| 10. | laser optics | 27. | reflectron |
| 11. | focused laser beam | 28. | detector |
| 12. | pixel | 29. | transient signal |
| 13. | residual walls | 30. | noise part |
| 14. | refinement part | 31. | individual peak |
| 15. | ICP-torch | 32. | data containing part |
| 16. | nebuliser gas inlet | 33. | 27Al |
| 17. | plasma beam | 34. | 69Ga |

## Claims

1. System (1) for handling gemstones (4), comprising a micro-inscribing system (2) for inscribing information (8) onto a surface part of a gemstone (4) using an ablation process, further comprising a material analysing system (3) for analysing the contents of a material composition, **characterised in that** the system (1) is designed in a way that the material that is set free by said micro-inscribing system (2) when inscribing information (8) onto a gemstone (4) is used by said material analysing system (3) for analysing the material composition of the gemstone (4).

2. System (1) according to claim 1, **characterised in that** the micro-inscribing system (2) is designed in a way to inscribe information, at least in part, in form of a series of points (12), in particular in form of a series of raster points (12).

3. System (1) according to claim 1 or 2, in particular according to claim 2, **characterised in that** said micro-inscribing system is designed in a way to inscribe information, at least in part, as a 2-D code (8), in particular as a data matrix code or a QR-code (8), and/or wherein the 2-D code (8) has a size of essentially and/or not more than 1 mm x 1 mm, preferably of essentially and/or not more than 0.5 mm x 0.5 mm, even more preferred of essentially and/or not more than 0.3 mm x 0.3 mm.

4. System (1) according to any of the preceding claims, in particular according to claim 3, **characterised in that** the system (1) further comprises an information generation system that is able to actuate said micro-inscribing system (2), in particular in a way that individual information (8) per gemstone (4) can be inscribed.

5. System (1) according to any of the preceding claims, **characterised in that** the micro-inscribing system (2) comprises a laser (9), in particular a laser (9) with a short wavelength of essentially and/or not more than 266 nm, 213 nm or 193 nm, and/or a focusing system (10) for focusing the outputted laser beam (11) of said laser (9), preferably a focusing system (10) for focusing the size of the laser beam spot to a size of 0.5 to 50 µm, 1 to 30 µm, 2 to 25 µm or 5 to 15 µm, preferably to essentially 10 µm.

6. System (1) according to any of the preceding claims, in particular system according to claim 5, **characterised in that** said laser is a pulsed laser (9), preferably a laser with a repetition rate of 1 to 100 Hz, preferably 5 to 50 Hz, even more preferred 10 to 25 Hz, in particular essentially 20 Hz and/or **characterised in that** said laser is a high-power laser (9) with a laser fluence of essentially and/or not less than 0.5 J/cm², preferably of essentially and/or not less than 1 J/cm², more preferred of essentially and/or not less than 2 J/cm², even more preferred of essentially and/or not less than 3 J/cm².

7. System (1) according to any of the preceding claims, **characterised in that** the material analysing system (3) comprises a mass spectrometry system (14, 24), preferably an inductively coupled plasma mass spectrometry system (14, 24), more preferred an inductively coupled plasma time-of-flight mass spectrometry system (14, 24) and/or an inductively coupled plasma quadrupole mass spectrometry system.

8. System (1) according to any of the preceding claims, preferably system according to claim 7, **characterised in that** said material analysing system (3) is designed in a way to analyse material that is preferably contained in a carrier gas (6, 7), wherein the said carrier gas is preferably an inert gas, taken from the group comprising helium, nitrogen, neon, argon, krypton or xeon.

9. Method for handling a gemstone (4), wherein the handling comprises at least a marking (8) of said gemstone (4) by an ablation process and an analysis of the material composition of said gemstone, **characterised in that** the material that is set free by the ablation process is used for analysing the material composition of the gemstone (4).

10. Method according to claim 9, **characterised in that** the ablating process is performed by a laser beam (11), preferably using a spotwise ablation of the gemstone (4), wherein preferably material that is ablated in a spotwise manner is used for analysing purposes.

11. Method according to claim 9 or 10, **characterised in that** a cleaning ablation step is performed prior to an analysis ablation step, where only the material that is ablated during the analysis ablation step is used for analysing the material composition of the gemstone (4).

12. Method according to any of claims 9 to 11, **characterised in that** the analysis of the material is at least in part performed by using mass spectrometry methods, in particular inductively coupled plasma mass spectrometry methods, preferably inductively coupled plasma time-of-flight mass spectrometry methods and/or inductively coupled plasma quadrupole mass spectrometry methods.

13. Method according to any of claims 9 to 12, **characterised in that** the marking of the gemstone (4), in particular the marking of the gemstone (4) in a spotwise manner, comprises a 2-D code, in particular a data matrix code or a QR-code (8).

14. Method according to any of claims 9 to 13, in particular according to claim 13, **characterised in that** the marking varies at least in part with the marked gemstone (4).

## Patentansprüche

1. System (1) zur Handhabung von Edelsteinen (4), umfassend ein Mikro-Beschriftungssystem (2) zum Einschreiben von Informationen (8) auf einem Flächenabschnitt von einem Edelstein (4), welches ein Ablationsverfahren verwendet, ferner umfassend ein Materialanalysesystem (3) zum Analysieren der Inhalte einer Materialzusammensetzung, **dadurch gekennzeichnet, dass** das System (1) derart ausgebildet ist, dass das Material, welches beim Einschreiben von Information (8) auf einem Edelstein (4) durch das Mikro-Beschriftungssystem (2) freigesetzt wird, von dem Materialanalysesystem (3) verwendet wird, um die Materialzusammensetzung von dem Edelstein (4) zu analysieren.

2. System (1) gemäss Anspruch 1, **dadurch gekennzeichnet, dass** das Mikro-Beschriftungssystem (2) ausgebildet ist, um Informationen einzuschreiben, zumindest teilweise in Form einer Serie von Punkten (12), insbesondere in Form einer Serie von Rasterpunkten (12).

3. System (1) gemäss Anspruch 1 oder 2, **dadurch gekennzeichnet, dass** das Mikro-Beschriftungssystem ausgebildet ist, um Informationen einzuschreiben, zumindest teilweise als ein 2-D Code (8), insbesondere als ein Daten-Matrix-Code oder ein QR-Code (8), und wobei der 2-D Code (8) eine Grösse von nicht mehr als 1 mm x 1 mm, bevorzugt von nicht mehr als 0.5 mm x 0.5 mm, mehr bevorzugt von nicht mehr als 0.3 mm x 0.3 mm aufweist.

4. System (1) gemäss einem der vorhergehenden Ansprüche, **dadurch gekennzeichnet, dass** das System (1) weiter ein Informationserzeugungssystem umfasst, welches das Mikro-Beschriftungssystem (2) aktivieren kann, insbesondere derart, dass individuelle Informationen (8) pro Edelstein (4) eingeschrieben werden können.

5. System (1) gemäss einem der vorhergehenden Ansprüche, **dadurch gekennzeichnet, dass** das Mikro-Beschriftungssystem (2) einen Laser (9) umfasst, insbesondere einen Laser (9) mit einer kurzen Wellenlänge von nicht mehr als 266 nm, 213 nm <sic. m> oder 193 nm, und ein Fokussiersystem (10) zum Fokussieren des ausgegebenen Laserstrahls (11) von dem Laser (9), bevorzugt ein Fokussiersystem (10) zum Fokussieren der Grösse des Laserstrahlpunkts auf eine Grösse von 0.5 bis 50 µm, 1 bis 30 µm, 2 bis 25 µm oder 5 bis 15 µm, bevorzugt auf 10 µm.

6. System (1) gemäss einem der vorhergehenden Ansprüche, insbesondere System gemäss Anspruch 5, **dadurch gekennzeichnet, dass** der Laser ein gepulster Laser (9) ist, bevorzugt ein Laser mit einer Wiederholungsrate von 1 bis 100 Hz, bevorzugt 5 bis 50 Hz, mehr bevorzugt 10 bis 25 Hz, insbesondere 20 Hz und/oder **dadurch gekennzeichnet, dass** der Laser ein Hochleistungs-Laser (9) ist mit einer Laserfluenz von nicht weniger als 0.5 J/cm², bevorzugt von nicht weniger als 1 J/cm², mehr bevorzugt von nicht weniger als 2 J/cm², noch mehr bevorzugt von nicht weniger als 3 J/cm² ist.

7. System (1) gemäss einem der vorhergehenden Ansprüche, **dadurch gekennzeichnet, dass** das Materialanalysesystem (3) ein Massenspektrometriesystem (14, 24) umfasst, bevorzugt ein induktiv gekoppeltes Plasma-Massenspektrometriesystem (14, 24), mehr bevorzugt ein induktiv gekoppeltes Plasma-Flugzeit-Massenspektrometriesystem (14, 24) und/oder ein induktiv gekoppeltes Plasma-Quadrupol-Massenpektrometriesystem.

8. System (1) gemäss einem der vorhergehenden Ansprüche, **dadurch gekennzeichnet, dass** das Materialanalysesystem (3) ausgebildet ist, um Material zu analysieren, welches in einem Trägergas (6, 7) enthalten ist, wobei das Trägergas bevorzugt ein Inertgas ist, entnommen der Gruppe, umfassend Helium, Stickstoff, Neon, Argon, Krypton oder Xeon.

9. Verfahren zur Handhabung eines Edelsteins (4), wobei die Handhabung zumindest eine Markierung (8) von dem Edelstein (4) durch ein Ablationsverfahren und eine Analyse der Materialzusammensetzung von dem Edelstein umfasst, **dadurch gekennzeichnet, dass** das Material, welches durch das Ablationsverfahren freigesetzt ist, zum Analysieren der Materialzusammensetzung von dem Edelstein (4) verwendet wird.

10. Verfahren gemäss Anspruch 9, **dadurch gekennzeichnet, dass** das Ablationsverfahren von einem Laserstrahl (11) ausgeführt wird, bevorzugt eine punktgenaue Ablation des Edelsteins (4), wobei Material, welches punktgenau abgetragen ist, für Analysezwecke verwendet wird.

11. Verfahren gemäss Anspruch 9 oder 10, **dadurch gekennzeichnet, dass** ein Reinigungsablationsschritt vor einem Analyseablationsschritt ausgeführt wird, wobei nur das Material, welches während des Analyseablationsschritts abgetragen wird, zum Analysieren der Materialzusammensetzung von dem Edelstein (4) verwendet wird.

12. Verfahren gemäss einem der Ansprüche 9 bis 11, **dadurch gekennzeichnet, dass** die Analyse des Materials zumindest teilweise ausgeführt wird mittels Verwendung von Massenspektrometrie-Verfahren, insbesondere induktiv gekoppelten Plasma-Massenspektrometrie-Verfahren, bevorzugt induktiv gekoppelten Plasma-Flugzeit-Massenspektrometrie-Verfahren und/oder induktiv gekoppelten Plasma-Quadrupol-Massenspektrometrie-Verfahren.

13. Verfahren gemäss einem der Ansprüche 9 bis 12, **dadurch gekennzeichnet, dass** die Markierung von dem Edelstein (4), insbesondere die Markierung von dem Edelstein (4) in einer punktgenauen Weise, einen 2-D Code, insbesondere einen Daten-Matrix-Code oder einen QR-Code (8) umfasst.

14. Verfahren gemäss einem der Ansprüche 9 bis 13, **dadurch gekennzeichnet, dass** die Markierung zumindest teilweise mit dem markierten Edelstein (4) variiert.

## Revendications

1. Système (1) pour le traitement des pierres précieuses (4), comprenant un système de micro-inscription (2) pour l'inscription d'informations (8) sur une partie de surface d'une pierre précieuse (4) en utilisant un procédé d'ablation, comprenant en outre un système d'analyse de matériau (3) pour analyser les contenus d'une composition de matériau, **caractérisé en ce que** le système (1) est conçu de manière que le matériau qui est libéré par ledit système de micro-inscription (2) lors de l'inscription des informations (8) sur une pierre précieuse (4) est utilisée par ledit système d'analyse de matériau (3) pour analyser la composition du matériau de la pierre précieuse (4).

2. Système (1) selon la revendication 1, **caractérisé en ce que** le système de micro-inscription (2) est conçu d'une manière à inscrire les informations, au moins en partie, sous la forme d'une série de points (12), en particulier sous la forme d'une série de points de trame (12).

3. Système (1) selon la revendication 1 ou 2, **caractérisé en ce que** ledit système de micro-inscription est conçu d'une manière à inscrire des informations, au moins en partie, comme un code 2D (8), en particulier comme un code data matrix ou un QR-code (8) et dans lequel le code 2D (8) a une taille de pas plus de 1 mm x 1 mm, de préférence de pas plus de 0.5 mm x 0.5 mm, de manière encore plus préférée de pas plus de 0.3 mm x 0.3 mm.

4. Système (1) selon l'une des revendications précédentes, **caractérisé en ce que** le système (1) comprend en outre un système de génération d'information qui est apte d'actionner ledit système de micro-inscription (2), en particulier d'une manière que des informations individuelles (8) par pierre précieuse (4) peuvent être inscrites.

5. Système (1) selon l'une des revendications précédentes, **caractérisé en ce que** le système de micro-inscription (2) comprend un laser (9), en particulier un laser (9) avec une longueur d'onde courte de pas plus de 266 nm, 213 nm ou 193 nm et un système de focalisation (10) pour la focalisation du faisceau laser (11) produit par ledit laser (9), préférablement un système de focalisation (10) pour focaliser la taille du point du faisceau laser à une taille de 0.5 à 50 µm, 1 à 30 µm, 2 à 25 µm ou 5 à 15 µm, préférablement de 10 µm.

6. Système (1) selon l'une des revendications précédentes, en particulier selon la revendication 5, **caractérisé en ce que** ledit laser est un laser pulsé (9), de manière préférée un laser avec une fréquence de récurrence de 1 à 100 Hz, préférablement 5 à 50 Hz, de manière encore plus préférée 10 à 25 Hz, en particulier 20 Hz et/ou **caractérisé en ce que** ledit laser est un laser haute puissance (9) avec une fluence de laser de pas moins de 0.5 J/cm², préférablement de pas moins de 1 J/cm², de manière plus préférée de pas moins de 2 J/cm², de manière encore plus préférée de pas moins de 3 J/cm².

7. Système (1) selon l'une des revendications précédentes, **caractérisé en ce que** le système d'analyse de matériau (3) comprend un système de spectrométrie de masse (14, 24), préférablement un système de spectrométrie de masse plasma couplé inductivement (14, 24), de manière plus préférée un système de spectrométrie de masse par temps de vol plasma couplé inductivement (14, 24) et/ou un système de spectrométrie de masse quadrupole plasma couplé inductivement.

8. Système (1) selon l'une des revendications précédentes, **caractérisé en ce que** ledit système d'analyse de matériau (3) est conçu de manière à analyser le matériau qui est contenu dans un gaz porteur (6, 7), dans lequel ledit gaz porteur est de préférence un gaz inerte, pris du groupe comprenant l'hélium, le nitrogène, le néon, l'argon, le krypton ou le xéon.

9. Procédé pour le traitement d'une pierre précieuse (4), dans lequel le traitement comprend au moins un marquage (8) de ladite pierre précieuse (4) par un procédé d'ablation et une analyse de la composition du matériau de ladite pierre précieuse, **caractérisé en ce que** le matériau qui est libéré par le procédé d'ablation est utilisé pour analyser la composition du matériau de la pierre précieuse (4).

10. Procédé selon la revendication 9, **caractérisé en ce que** le procédé d'ablation est effectué par un faisceau laser (11), en utilisant de préférence une ablation ponctuelle de la pierre précieuse (4), dans lequel le matériau qui est enlevé d'une manière ponctuelle est utilisé à des fins d'analyse.

11. Procédé selon la revendication 9 ou 10, **caractérisé en ce qu'**une étape d'ablation de nettoyage est réalisée avant l'étape d'ablation d'analyse, où seulement le matériau qui est enlevé pendant l'étape d'ablation d'analyse est utilisée pour analyser la composition du matériau de la pierre précieuse (4).

12. Procédé selon l'une des revendications 9 à 11, **caractérisé en ce que** l'analyse du matériau est au moins en partie réalisée en utilisant des procédés de spectrométrie de masse, en particulier des procédés de spectrométrie de masse plasma couplé inductivement, de manière préférée des procédés de spectrométrie de masse par temps de vol plasma couplé inductivement et/ou des procédés de spectrométrie de masse quadrupole plasma couplé inductivement

13. Procédé selon l'une des revendications 9 à 12, **caractérisé en ce que** le marquage de la pierre précieuse (4), en particulier le marquage de la pierre précieuse (4) d'une manière ponctuelle, comprend un code 2D, en particulier un code data matrix ou un QR-code (8).

14. Procédé selon l'une des revendications 9 à 13, **caractérisé en ce que** le marquage varie au moins en partie avec la pierre précieuse marquée (4).
